# FASCICULE DE BREVET EUROPEEN

(11) **EP 0 545 085 B1**
(45) Date de publication et mention de la délivrance du brevet: **01.03.2000**
(21) Numéro de dépôt: 92118834.8
(22) Date de dépôt: 04.11.1992
(51) Int. Cl.: C07D 207/20, A23L 1/226

(54) **Procédé d'obtention d'acétyl-2-pyrroline-1 sous forme encapsulée, composé intermédiaire et produit final obtenus**
Verfahren zur Herstellung von 2-Acetyl-1-Pyrrolin in verkapselter Form, Zwischenprodukte und Endprodukt
Process for obtaining 2-acetyl-1-pyrroline in encapsulated form, intermediates and final product

(30) Priorité: 02.12.1991 CH 352991
(43) Date de publication de la demande: 09.06.1993
(73) Titulaire: SOCIETE DES PRODUITS NESTLE S.A., 1800 Vevey (CH)
(72) Inventeur: Duby, Philippe, CH-1008 Prilly (CH); Huynh-Ba, Tuong, CH-1009 Pully (CH)
(74) Mandataire: Vuille, Roman

(56) Documents cités:
- EP-A- 0 436 481
- US-A- 4 522 838
- CHEMICAL ABSTRACTS, vol. 110, 1989, Columbus, Ohio, US; abstract no. 74119, SCHIEBERLE, P. 'Effect of the addition of proline on the formation of the flavour compound 2-acetyl-1-pyrroline in the crust of white bread.'
- CHEMICAL ABSTRACTS, vol. 99, 1983, Columbus, Ohio, US; abstract no. 69010, BUTTERY R. G. ET. AL. 'Identification of rice aroma compound 2-acetyl-1-pyrroline in pandan leaves.'
- CHEMICAL ABSTRACTS, vol. 98, 1983, Columbus, Ohio, US; abstract no. 196668, BUTTERY R. G. ET. AL. '2-ACETYL-1-PYRROLINE: AN IMPORTANT AROMA COMPONENT OF COOKED RICE'
- CHEMICAL ABSTRACTS, vol. 99, 1983, Columbus, Ohio, US; abstract no. 37298, BUTTERY R. G. ET. AL. 'Cooked rice aroma and 2-acetyl-1-pyrroline.'
- G. DITTUS, H. KRÖPER, H. MEERWEIN 'Methoden der Organischen Chemie, Band VI/3 Sauerstoffverbindungen' 1965 , GEORG THIEME VERLAG , STUTTGART

## Description

La présente invention concerne un procédé d'obtention d'acétyl-2-pyrroline-1 se présentant sous forme encapsulée, le produit encapsulé obtenu et une utilisation particulière de ce produit. La présente invention concerne également un nouveau composé de la famille des pyrrolines, permettant l'obtention d'acétyl-2-pyrroline-1 encapsulée, son procédé de préparation et une utilisation particulière.

L'acétyl-2-pyrroline a été identifiée comme une composante de l'arôme de riz cuit, par exemple, dans les documents Chem. Abs. **99**, 1983, 69010 ; Chem. Abs. **98,** 1983, 196668 et Chem. Abs. **99** 1983, 37298y. Aussi, le document Chem. Abs. **110**, 1989, 74119 décrit l'effet de l'addition de proline sur la formation de ce composé aromatisant dans la croûte du pain blanc.

Le brevet US-A-4,522,838 de Buttery et al décrit un procédé de préparation de l'acétyl-2-pyrroline-1. Ce procédé consiste en une réduction catalytique de l'acétyl-2-pyrrole sous une pression d'hydrogène d'environ 0,7 bar pendant 15 heures, de manière à obtenir comme produit intermédiaire l'(hydroxyéthyl-1)-2-pyrrolidine, suivie d'une oxydation dudit produit intermédiaire avec du carbonate d'argent, sous reflux dans le benzène, pendant encore 15 heures. L'acétyl-2-pyrroline-1 est ensuite isolé par chromatographie en phase gazeuse à l'aide d'une colonne capillaire de 2 mètres de long et de 6,4 mm de diamètre.
L'acétyl-2-pyrroline-1 ainsi obtenue présente une pureté de 95% environ, mais ne peut être préparée qu'en petites quantités à la fois, ceci étant dû principalement à l'étape de purification du composé, effectuée par chromatographie en phase gazeuse. De plus, l'acétyl-2-pyrroline-1 est peu stable et doit de préférence être conservée à une température inférieure à 0°C, ce qui rend son emploi difficile, particulièrement au niveau industriel.

On connaît aussi la demande de brevet EP-A-436 481 qui concerne un autre procédé de préparation de l'acétyl-2-pyrroline-1 à partir de la pyrrolidine, mais qui ne résoud pas ce problème.

La présente invention concerne un procédé permettant d'obtenir de l'acétyl-2-pyrroline-1 se présentant sous forme stable, et pouvant être conservée pendant un certain temps à température ambiante.

La présente invention a donc pour premier objet l'acétyl-2-pyrroline-1 se présentant sous forme encapsulée susceptible d'être obtenue par le procédé dans lequel, à une température de -10°C à 25°C, on hydrolyse l'(éthoxy-1-éthenyl)-2-pyrroline -1 avec un acide, on neutralise avec une quantité équimolaire de base, on ajoute une solution de cyclodextrine de manière à obtenir une solution contenant jusqu'à 20% en poids d'acétyl 2-pyrroline-1 par rapport à la cyclodextrine, puis on lyophilise ladite solution de manière à obtenir une poudre pouvant contenir jusqu'à 20% en poids d'acétyl-2-pyrroline-1.

La présente invention a pour second objet l'acétyl-2-pyrroline-1 se présentant sous forme encapsulée susceptible d'être obtenue par le procédé dans lequel, à une température de -10°C à 25°C, on hydrolyse l'(éthoxy-1-éthenyl)-2-pyrroline -1 avec un acide, on neutralise avec une quantité équimolaire de base, on ajoute une solution de maltodextrine de manière à obtenir une solution contenant jusqu'à 10% en poids d'acétyl-2 -pyrroline-1 par rapport à la maltodextrine, puis on lyophilise ladite solution de manière à obtenir une poudre pouvant contenir jusqu'à 10% en poids d'acétyl-2-pyrroline-1.

Il est ainsi possible de produire en grande quantité, et de manière industrielle, de l'acétyl-2-pyrroline-1 se présentant sous forme stable, dont la conservation et/ou l'utilisation sont beaucoup plus aisées par comparaison avec de l'acétyl-2-pyrroline-1 presque pure.

De plus, le produit ainsi obtenu peut également être utilisé dans le domaine alimentaire, comme exhausteur de goût.

Le produit, premier objet de l'invention, est susceptible d'être obtenu par le procédé dans lequel, on hydrolyse l'(éthoxy-1- éthenyl)-2-pyrroline-1, de préférence en solution à une concentration de 2 à 30%, avec un acide, de préférence un acide minéral tel que l'acide chlorhydrique. On peut alors diluer le mélange obtenu, puis on neutralise l'acide ajouté par addition d'une quantité équimolaire de base, de préférence une base forte telle que l'hydroxyde de sodium. On ajoute alors audit mélange neutralisé, une solution de cyclodextrine ou de maltodextrine. On ajoute de préférence une solution de β-cyclodextrine. Il est également possible d'ajouter une solution contenant une ou plusieurs cyclodextrines et de la maltodextrine.

On ajoute de la cyclodextrine en une quantité telle que l'on obtient ensuite une solution contenant jusqu'à 20% d'acétyl-2-pyrroline-1 par rapport à ladite cyclodextrine. Si l'on utilise de la maltodextrine, on l'ajoute en une quantité telle que l'on obtient ensuite une solution contenant jusqu'à 10% d'acétyl-2-pyrroline-1 par rapport à ladite maltodextrine.

Les différentes étapes de ce procédé sont effectuées à une température allant de -10°C à 25°C, étant donné que plus la température est élevée, plus les risques de décomposition de l'acétyl-2-pyrroline-1 sont élevés et plus il est nécessaire alors de travailler rapidement si l'on veut diminuer ces risques.
La solution ainsi obtenue est alors lyophilisée et permet l'obtention d'un produit encapsulé se présentant sous forme d'une poudre et pouvant contenir jusqu'à 20% en poids d'acétyl-2-pyrroline-1.

On a de plus constaté que, de manière surprenante, le produit encapsulé ainsi obtenu pouvait être utilisé pour renforcer le goût de certains produits alimentaires. La présente invention a donc également pour objet l'utilisation de ce produit encapsulé comme exhausteur de goût. Ainsi que le montreront les exemples, ce produit peut être utilisé seul ou en combinaison avec d'autres composés.

Afin de préparer l'acétyl-2-pyrroline-1 encapsulée selon l'un des procédés décrits ci-dessus, on utilise comme produit de départ l'(éthoxy-1-éthenyl)-2-pyrroline-1.
La présente invention a donc également pour objet l'(éthoxy-1-éthenyl)-2-pyrroline-1, de formule : 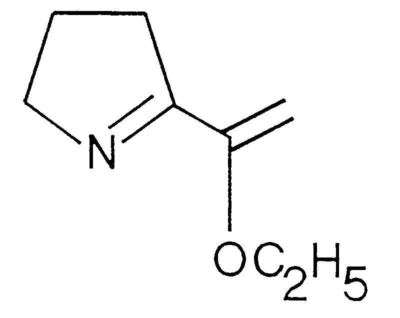 Ces composés peuvent être obtenus par un procédé dans lequel on fait réagir le composé alkylvinyléther correspondant avec du tert-butyllithium en solution organique, par exemple dans le pentane, le tétrahydrofurane ou l'éther, ou un mélange de ces solvants, puis on ajoute du N-triméthylsilylpyrrolidinone-2 et on laisse réagir, de préférence en dessous de 0°C, puis on hydrolyse la solution obtenue, on récupère la phase organique que l'on sèche et purifie de manière à obtenir l'(éthoxy-1-éthenyl)-2-pyrroline-1 recherchée.
L'hydrolyse est de préférence effectuée par addition d'eau ou d'une solution de chlorure d'ammoniaque. Après hydrolyse, il est possible d'ajouter à la solution un sel, tel que le chlorure de sodium, de manière à saturer ladite solution, ce qui permet d'améliorer le rendement du présent procédé.

La purification finale peut être effectuée par tout moyen tel que la distillation et/ou la chromatographie sur colonne. Ce procédé permet donc l'obtention de l'(éthoxy1-éthenyl)-2-pyrroline-1, qui peut servir à l'obtention de l'acétyl-2-pyrroline-1 encapsulée.
On a de plus constaté qu'un des composés de ce type,l'(éthoxy-1-éthenyl)-2-pyrroline-1 présentait certaines qualités organoleptiques qui permettaient de l'utiliser, seule ou en combinaison, comme agent aromatisant.
Ces caractéristiques organoleptiques peuvent être décrites ainsi: grillé, fruité, noisettes, et rappellent celles de la pyrazine.
Ainsi, l'(éthoxy-1-éthenyl)-2-pyrroline-1 peut être utilisée, seule, comme agent aromatisant, de manière à remplacer, par exemple, certaines alkyl-pyrazines.
La quantité à ajouter à un produit alimentaire dépend principalement de la nature du produit ainsi que de l'effet final souhaité.

L'(éthoxy-1-éthenyl)-2-pyrroline-1 peut également être utilisée dans l'élaboration d'une composition aromatisante. On peut par exemple préparer une composition aromatisante contenant jusqu'à 0,1% en poids d'(éthoxy-1-éthenyl)-2-pyrroline-1 et présentant un arôme de maïs, pain ou de pommes-de-terre. La composition ainsi obtenue peut être ajoutée au produit alimentaire final, à raison de 1 à 2 grammes par kilo de produit final.
Un autre objet de l'invention est donc l'utilisation de l'(éthoxy-1-éthenyl)-2-pyrroline-1 comme agent aromatisant susceptible d'être ajouté à un produit alimentaire.
Encore un autre objet de l'invention est l'utilisation de l'(éthoxy-1-éthenyl)-2-pyrroline-1 dans une composition aromatisante susceptible d'être ajoutée à un produit alimentaire. Enfin, un dernier objet de l'invention est une composition aromatisante comprenant l'(éthoxy-1-éthenyl)-2-pyrroline-1.

L'invention est illustrée plus en détail dans les exemples suivants, dans lesquels les parties et pourcentages sont en poids:
- l'exemple 1 décrit un procédé de préparation de l'(éthoxy-1-éthenyl)-2-pyrroline-1,
- l'exemple 2 décrit les tests d'identification de ce composé,
- l'exemple 3 illustre les propriétés aromatisantes de ce composé,
- les exemples 4 et 5 illustrent deux procédés d'obtention d'acétyl-2-pyrroline-1 en solution aqueuse,
- les exemples 6-9 illustrent trois procédés d'obtention d'acétyl-2-pyrroline-1 se présentant sous forme encapsulée et le produit obtenu,
- l'exemple 10 illustre la stabilité au stockage de l'acétyl-2-pyrroline-1 encapsulée,
- les exemples 11-16 illustrent les propriétés de l'acétyl-2-pyrroline-1 encapsulée, seule ou dans une composition exhausteur de goût.

### Exemple 1

On prépare une solution contenant 54,1 g d'éthylvinyléther dans 300 ml de tétrahydrofurane (THF).

On refroidit cette solution à -40°C et on lui ajoute, goutte-à-goutte, une solution contenant 429 ml de tert-butyllithium à 1,4 N dans le pentane.
On laisse ce mélange sous agitation constante, à -40°C, pendant 12 heures, puis on lui ajoute une solution de 47,1 g de N-triméthylsilyl-pyrrolidinone-2 dans 300 ml de THF.
Le mélange ainsi obtenu est laissé sous agitation constante à -40°C pendant 7 heures, puis on lui ajoute 32,1 g de chlorure d'ammonium dissout dans 300 ml d'eau.
On laisse le mélange se réchauffer jusqu'à environ 0°C, puis on le sature par addition de 60 g de chlorure de sodium.
On laisse reposer afin que les phases aqueuse et organique se séparent.
On récupère la phase organique, et l'on extrait la phase aqueuse trois fois avec 50 ml d'éther.

On mélange les différentes fractions organiques obtenues et on les lave trois fois avec 50 ml d'eau saturée avec NaCl, puis on les sèche sur du sulfate de sodium et l'on évapore les solvants sous pression réduite.
On obtient 37,8 g d'extrait brut se présentant sous forme d'un liquide jaune.
On prépare une colonne de chromatographie d'un diamètre de 4 cm, d'une hauteur de 80 cm et contenant 500 g d'un gel silicagel.
On utilise comme éluant une solution contenant 20 parties de dichlorométhane pour 1 partie d'acétate d'éthyle.
On introduit dans la colonne l'extrait brut dilué avec de l'éluant, puis on l'élue à un débit de 2 ml par minute.
On obtient alors 17,1 g d'un composé possédant une pureté de 99,9%, se présentant sous forme d'un liquide incolore.

### Exemple 2

### 1. Spectre de masse

Le spectre de masse du composé obtenu selon l'exemple 1 donne les résultats suivants:

| m/z | intensité relative (100=pic de base) | identification |
|---|---|---|
| 139 | 8 | [M]+ pic moléculaire |
| 124 | 21 | [M-CH₃]+ |
| 110 | 5 | [M-C₂H₅]+ |
| 95 | 100 | [M-CH₃CHO]+ |
| 94 | 66 | [M-OC₂H₅]+ |
| 83 | 6 | --- |
| 82 | 10 | --- |
| 67 | 18 | --- |
| 41 | 30 | --- |

### 2. Analyse élémentaire

L'analyse élémentaire du composé obtenu selon l'exemple 1, effectuée par combustion, donne les résultats suivants:

| | | % |
|---|---|---|
| C | mesuré | 68,70 |
| | calculé | 69,03 |
| H | mesuré | 9,26 |
| | calculé | 9,41 |
| N | mesuré | 9,74 |
| | calculé | 10,06 |

Les résultats calculés le sont pour le composé de formule brute C₈H₁₃NO, de poids moléculaire M=139,198g.

### 3. Spectre infrarouge

Le spectre infrarouge du composé obtenu selon l'exemple 1, effectué à l'état de film donne les résultats suivants:

| Fréquence des bandes caractéristiques (cm⁻¹) | % de transmission |
|---|---|
| 2977 | 23,8 |
| 2932 | 28,9 |
| 2862 | 34,5 |
| 1738 | 45,8 |
| 1612 | 26,9 |
| 1591 | 20,5 |
| 1370 | 29,7 |
| 1322 | 19,7 |
| 1271 | 25,1 |
| 1129 | 20,6 |
| 1068 | 25,6 |
| 979 | 34,7 |
| 819 | 30,2 |

### 4. Spectre RMN

Le spectre de résonance magnétique nucléaire du proton du composé dans le trichlorodeutérométhane (CDCl₃) à 20°C, montre les signaux caractéristiques suivants:

| Signal (ppm) | Multiplicité du signal | Constante de couplage (Hz) | Identification |
|---|---|---|---|
| 4,73 | doublet | 2,6 | H oléfinique |
| 4,52 | doublet | 2,6 | H oléfinique |
| 4,02 | triplet d'un triplet | 2,0 et 7,4 | 2H-5 |
| 3,87 | quadruplet | 7 | 2H d'éthyl |
| 2,73 | multiplet | | 2H-3 |
| 1,93 | multiplet | | 2H-4 |
| 1,42 | triplet | 7 | 3H d'éthyl |

A l'aide de ces quatre tests, on peut donc identifier le composé comme étant l'(éthoxy-1-éthenyl)-2-pyrroline-1, de formule:

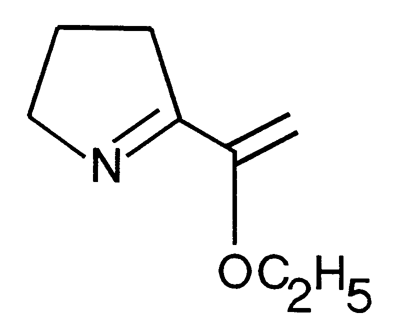

### Exemple 3

### a) Détection du seuil de perception par voie olfactive directe

On prépare plusieurs solutions aqueuses contenant de l'(éthoxy-1-éthenyl)-2-pyrroline-1 à différentes concentrations.

On présente 50 ml de chaque solution, en erlenmeyer de 250 ml recouvert d'un couvercle, à 6 dégustateurs entraînés à l'analyse des arômes.
Le test se déroule de la manière suivante:
on présente 3 flacons à chaque dégustateur: un flacon contenant la solution aromatique et deux flacons d'eau. Le dégustateur doit humer l'espace de tête et désigner le flacon contenant la solution aromatique.
Le test est répété deux fois, pour des concentrations de 1000, 100, 10, 5 et 1 ppm.

On obtient les résultats suivants:

| | | | | | |
|---|---|---|---|---|---|
| concentration (ppm) | 1000 | 100 | 10 | 5 | 1 |
| olfaction positive(%) | 100 | 100 | 100 | 50 | 17 |

Le seuil de perception par voie olfactive directe est donc de l'ordre de 5 ppm.

### b) Détection du seuil de perception par GC-sniffing

On détermine par "GC-sniffing" (combinaison de chromatographie gazeuse et olfactométrie) la quantité minimale d'(éthoxy-1-éthenyl)-2-pyrroline-1 détectable olfactivement.
Pour ce faire, on analyse par chromatographie en phase gazeuse des solutions du composé à différentes concentrations.

Après des tests d'orientation, des solutions d'(éthoxy-1-éthenyl)-2-pyrroline-1 dans le dichlorométhane sont préparées avec une concentration de 100, 500 et 1000 ppm.
Les analyses sont réalisées selon les conditions suivantes:
. Chromatographe HP 5890 A (Hewlett-Packard)
. Colonne Capillaire DB-Wax (J et W Scientific), longueur 30 m, i.d. = 0.25 mm
. Détection FID, 250°C + Sniffing Port, 150°C Effluent splitting: 1/1
. Injection 200°C, Split (27.7 ml/min)
. Gaz vecteur Helium, 17.5 psi
. Température four 100°C - 4°/min - 180°C
. Quantité injectée: 1 microlitre d'une solution à 1000 ppm, soit 1 microgramme du composé.
. Débits gazeux: split vent = 27.7 ml/min effluent colonne = 0.6 ml/min (sniffing port)
. Quantité (éthoxy-1-éthenyl)-2-pyrroline-1 arrivant au sniffing port = 21.7 ng
. Estimation du volume d'air entraînant l'(éthoxy-1-éthenyl)-2-pyrroline-1
. Durée du pic olfactif = 5 secondes
. Débit d'air humidifié (make-up) = 0.83 ml/sec
. Volume d'air estimé à 4.2 ml d'air
Le seuil de perception de l'(éthoxy-1-éthenyl)-2-pyrroline-1 est de 21,7 ng dans 4,2 ml d'air soit 5,20 ng/ml air.

### Exemple 4

On effectue une hydrolyse de l'(éthoxy-1-éthenyl)-2-pyrroline-1, en ajoutant à 67,74 mg d'(éthoxy-1-éthenyl)-2-pyrroline-1 à 0°C, 5ml d'acide chlorhydrique 10,5N; on laisse reposer à température ambiante (25°C) pendant 2 jours.

On refroidit ensuite le mélange jusqu'à -5°C environ, et on le neutralise, en ajoutant goutte-à-goutte, et sous agitation constante, 52,5 ml d'hydroxyde de sodium 1N.
On obtient alors une solution aqueuse contenant 97% d'acétyl-2-pyrroline-1, soit 52,40 mg, et 3% d'acétyl-2-pyrroline-2, soit 1,62 mg (composition déterminée par chromatographie en phase gazeuse et spectrométrie de masse).

### Exemple 5

On effectue une hydrolyse de l'(éthoxy-1-éthenyl)-2-pyrroline-1 en ajoutant à 69,81 mg d'(éthoxy-1-éthenyl)-2-pyrroline-1 à 0°C, 5 ml d'acide chlorhydrique 1N; on laisse alors reposer à température ambiante (25°C) pendant 7 jours, afin que l'hydrolyse soit complète.

On refroidit ensuite le mélange jusqu'à -5°C environ, on dilue le mélange hydrolysé avec 40 ml d'eau, puis on le neutralise en ajoutant 5 ml d'hydroxyde de sodium 1 N, goutte-à-goutte, sous agitation constante.

On obtient une solution aqueuse contenant 54 mg d'acétyl-2-pyrroline-1 (97%) et 1,67 mg d'acétyl-2-pyrroline-2 (3%).

### Exemple 6

On ajoute à 1,00132 g d'(éthoxy-1-éthenyl)-2-pyrroline-1 (soit 7,20 mmol) à 0°C, 70 ml d'HCl 1N, et on laisse reposer à température ambiante pendant 7 jours.

Un premier échantillon A de 17,5 ml de ce mélange réactionnel est dilué dans 175 ml d'eau, puis refroidit à 0°C. On lui ajoute alors goutte-à-goutte 17,5 ml de NaOH 1N, puis une solution aqueuse comprenant 17,6 g de maltodextrine, 2,4 g de gomme arabique et 430 ml d'eau.
La solution ainsi obtenue est lyophilisée dans un lyophilisateur standard. On obtient une poudre blanche contenant l'acétyl-2-pyrroline-1 à la concentration de 1,0 % par rapport au mélange maltodextrine/gomme arabique, et à la concentration de 0,94% par rapport à la poudre.

### Exemple 7

Un échantillon B de 13,125 ml du mélange réactionnel obtenu selon l'exemple 6 est dilué dans 130 ml d'eau, puis refroidit à 0°C. On lui ajoute goutte-à-goutte 13,125 ml de NaOH 1N, puis une solution aqueuse comprenant 15 g de β-cyclodextrine dans 400 ml d'eau.
La solution ainsi obtenue est lyophilisée selon l'exemple 6. On obtient une poudre blanche contenant l'acétyl-2-pyrroline-1 à la concentration de 1,0 % par rapport à la β-cyclodextrine, et de 0,94% par rapport à la poudre.

### Exemple 8

Un échantillon C de 13,125 ml du mélange réactionnel obtenu selon l'exemple 6 est dilué dans 100 ml d'eau, puis refroidit à 0°C.
On lui ajoute 15,0 g de β-cyclodextrine en poudre et 13,125 ml de NaOH 1N. On laisse sous agitation à 0°C pendant 30 minutes, puis la solution est lyophilisée selon l'exemple 6.
Dans ce cas, la lyophilisation est plus aisée à effectuer car le volume d'eau est beaucoup moins important, par comparaison avec les exemples 6 et 7.

On obtient une poudre blanche contenant l'acétyl-2-pyrroline-1 à la concentration de 1,0 % par rapport à la β-cyclodextrine, et de 0,94% par rapport à la poudre.

### Exemple 9

Un échantillon D de 13, 125 ml du mélange réactionnel obtenu selon l'exemple 6 est dilué dans 130 ml d'eau, puis refroidit à 0°C. On lui ajoute goutte-à-goutte 13,125 ml de NaOH 1N puis une solution aqueuse comprenant 1,5 g de β-cyclodextrine dans 40 ml d'eau.
La solution ainsi obtenue est lyophilisée selon l'exemple 6. On obtient une poudre blanche contenant l'acétyl-2-pyrroline-1 à la concentration de 10,0 % par rapport à la β-cyclodextrine.

### Exemple 10

On étudie la stabilité de l'acétyl-2-pyrroline-1 encapsulée préparée selon les exemples 6 à 9, pendant une période de 110 jours, à différentes températures de stockage.
Pour ce faire, on prélève, après stockage, 100 mg de poudre lyophilisée, que l'on dissout dans 1 ml d'eau, à 0°C.
On ajoute 1 ml d'acétate d'éthyle contenant 1 mg de triméthyl-2,4,6-pyridine et on laisse sous agitation pendant 30 secondes.
On centrifuge le mélange pendant 15 minutes, à -5°C et l'on récupère la phase organique que l'on analyse par chromatographie en phase gazeuse.

On détermine alors le pourcentage d'acétyl-2-pyrroline-1 s'étant décomposée après 110 jours de stockage, et l'on obtient les résultats suivants:

| Support et concentration en acétyl-2-pyrroline-1 | | | |
|---|---|---|---|
| Température de stockage | maltodextrine 1% | β-cyclodextrine 1% | β-cyclodextrine 10% |
| 20°C | décomposition totale après 50j. | 99% | 91% après 13 jours |
| 4°C | 33% | 10% | 13% après 23 jours |
| - 20°C | 13% | 0% | --- |

On constate donc que l'acétyl-2-pyrroline-1 encapsulée à 1% sur β-cyclodextrine est stable pendant au moins 110 jours si on la conserve à une température basse. Ceci est également le cas, mais dans une moindre mesure, si l'encapsulation est effectuée sur maltodextrine/gomme arabique à 1%.
Par contre, la stabilité de l'acétyl-2-pyrroline-1 diminue quand sa concentration sur le support (dans le cas présent la β-cyclodextrine) augmente. Par comparaison, l'acétyl-2-pyrroline-1 à 95% pure, préparée selon l'art antérieur se dégrade rapidement conservée à -20°C (Buttery et al., Journal of Agric. Food Chem. (1983), 31, 823-826).

### Exemple 11

On ajoute 1ppm d'acétyl-2-pyrroline-1, sous forme encapsulée à 1% sur β-cyclodextrine, à une soupe de maïs, juste avant sa consommation. On présente la soupe ainsi préparée, ainsi qu'une soupe-témoin ne contenant pas d'acétyl-2-pyrroline-1, à un groupe de 6 dégustateurs entraînés.
L'acétyl-2-pyrroline-1 contribue à une perception plus ronde, plus cuisinée et plus flatteuse de l'aliment. Les notes "céréale cuite", "pop corn" et "très légèrement grillé" sont renforcées et mises en valeur, ainsi qu'une très fine note "beurré-frais".

### Exemple 12

On ajoute 1ppm d'acétyl-2-pyrroline-1 sous forme encapsulée à 1% sur β-cyclodextrine, à une sauce de type poulet, juste avant sa consommation. On présente la soupe ainsi préparée, ainsi qu'une soupe-témoin ne contenant pas d'acétyl-2-pyrroline-1, à un groupe de 6 dégustateurs entraînés.
L'acétyl-2-pyrroline-1 contribue à diminuer la note "graisse de poulet" et renforce les notes "chair de poulet" et "légèrement grillé".
L'ensemble est plus cuisiné et plus complet, et l'arrière-goût légèrement viande grillée est prolongé.

### Exemple 13

On ajoute 1ppm d'acétyl-2-pyrroline-1 sous forme encapsulée à 1% sur β-cyclodextrine, à une sauce de type boeuf, juste avant sa consommation. On présente la soupe ainsi préparée, ainsi qu'une soupe-témoin ne contenant pas d'acétyl-2-pyrroline-1, à un groupe de 6 dégustateurs entraînés.
L'acétyl-2-pyrroline-1 contribue à renforcer la note "viande légèrement grillée". L'impression en bouche est plus ronde et l'arrière-goût boeuf est prolongé.

### Exemple 14

On prépare une composition exhausteur de goût de type "croûte de pain" en ajoutant à 1 litre d'éthanol les composées suivants:
- 50 g d'acétyl-2-pyrazine
- 10 g d'acétyl-2-thiazole
- 30 g de diacétyle
- 5 g d'éthyl-2-méthyl-3-pyrazine

On ajoute 0,1 g de cette composition à 1 litre d'eau préalablement salée à raison de 3 g de NaCl par litre.
On divise le mélange aqueux en deux lots.
Dans le premier lot, on ajoute 0,5 ppm d'acétyl-2-pyrroline-1 sous forme encapsulée à 1% sur β-cyclodextrine.
Le second lot sert de témoin.
Un panel de 10 personnes compare les deux lots.
Le premier lot apparaît meilleur que le second, avec une note renforcée de type "céréale", "croûte de pain" et une note "grillée" arrondie. L'ensemble reste en bouche plus longtemps. Ajoutée à une pâte à pizza, la présente composition renforce la note "croûte de pain", surtout à l'olfaction.

### Exemple 15

On prépare une composition exhausteur de goût de type "maïs" en ajoutant à 1 litre d'éthanol les composées suivants:
- 5 g d'acétyl-2-pyrazine
- 5 g d'acétyl-2-thiazole
- 0,5 g de diacétyle
- 20 g de sulfure de diméthyle

On ajoute 0,1 g de cette composition à 1 litre d'eau préalablement salée à raison de 3 g de NaCl par litre.
On divise le mélange aqueux en deux lots.
Dans le premier lot, on ajoute 0,5 ppm d'acétyl-2-pyrroline-1 sous forme encapsulée à 1% sur β-cyclodextrine.
Le second lot sert de témoin.
Un panel de 10 personnes compare les deux lots.
Le premier lot présente une note plus typée, plus complète et plus puissante de "maïs doux" et "pop-corn".
La persistance en bouche est plus marquée.

### Exemple 16

On prépare une composition exhausteur de goût de type "pommes-de-terre" en ajoutant à 1 litre d'éthanol les composées suivants:
- 5 g d'acétyl-2-thiazole
- 5 g de triméthyl-pyrazine
- 0,5 g de diacétyle
- 2 g d'éthyl-2-méthoxy-3-pyrazine
- 50 g de méthylthio-3-propanal

On ajoute 0,1 g de cette composition à 1 litre d'eau préalablement salée à raison de 3 g de NaCl par litre.
On divise le mélange aqueux en deux lots.
Dans le premier lot, on ajoute 0,5 ppm d'acétyl-2-pyrroline-1 sous forme encapsulée à 1% sur β-cyclodextrine.
Le second lot sert de témoin.
Un panel de 10 personnes compare les deux lots.
Le premier lot apparaît meilleur que le second et présente une note plus complète et renforcée de type "chair cuite de pommes-de-terre".

## Revendications

1. (Ethoxy-1-éthenyl)-2-pyrroline-1 de formule :

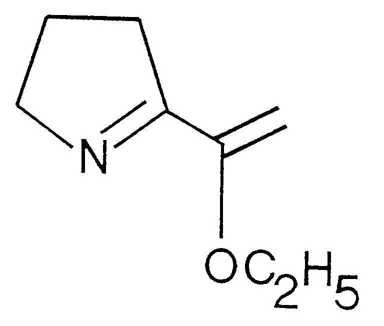

2. Procédé de préparation d'(éthoxy-1-éthenyl)-2-pyrroline-1 selon la revendication 1, dans lequel on fait réagir à une température inférieure à 0°C de l'éthylvinyléther et du tert-butyllithium en solution organique, on ajoute du N-triméthylsilyl-pyrrolidinone-2, on laisse réagir puis on hydrolyse la solution obtenue, on récupère la phase organique que l'on sèche et purifie afin d'obtenir l'(ethoxy-1-éthenyl)-2-pyrroline-1 recherchée.

3. Utilisation de l'(éthoxy-1-éthenyl)-2-pyrroline-1 selon la revendication 3 comme agent aromatisant susceptible d'être ajouté à un produit alimentaire.

4. Composition aromatisante comprenant l'(éthoxy-1-éthenyl)-2-pyrroline-1.

5. Acétyl-2-pyrroline-1 se présentant sous forme encapsulée susceptible d'être obtenue par le procédé dans lequel, à une température de -10°C à 25°C, on hydrolyse l'(éthoxy-1-éthenyl)-2-pyrroline-1 avec un acide, on neutralise avec une quantité équimolaire de base, on ajoute une solution de cyclodextrine de manière à obtenir une solution contenant jusqu'à 20% en poids d'acétyl-2-pyrroline-1 par rapport à la cyclodextrine, puis on lyophilise ladite solution de manière à obtenir une poudre pouvant contenir jusqu'à 20% en poids d'acétyl-2-pyrroline-1.

6. Acétyl-2-pyrroline-1 se présentant sous forme encapsulée susceptible d'être obtenue par le procédé dans lequel, à une température de -10°C à 25°C, on hydrolyse l'(éthoxy-1-éthenyl)-2-pyrroline-1 avec un acide, on neutralise avec une quantité équimolaire de base, on ajoute une solution de maltodextrine de manière à obtenir une solution contenant jusqu'à 10% en poids d'acétyl-2-pyrroline-1 par rapport à la maltodextrine, puis on lyophilise ladite solution de manière à obtenir une poudre pouvant contenir jusqu'à 10% en poids d'acétyl-2-pyrroline-1.

7. Utilisation du produit selon les revendications 5 et 6, seul ou en combinaison, comme exhausteur de goût, susceptible d'être ajouté à un produit alimentaire.

## Claims

1. (Ethoxy-1-ethenyl)-2-pyrroline-1 of formula:

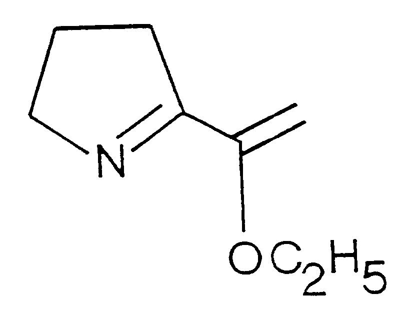

2. Method for preparing (ethoxy-1-ethenyl)-2-pyrroline-1 according to claim 1, wherein ethylvinylether and tert-butyllithium are reacted at a temperature below 0°C in organic solution, N-trimethylsilyl-pyrrolidinone-2 is added, the solution obtained is allowed to react and is then hydrolysed, and the organic phase is recovered which is dried and purified so as to obtain the desired (ethoxy-1-ethenyl)-2-pyrroline-1.

3. Use of (ethoxy-1-ethenyl)-2-pyrroline-1 according to claim 3 as a flavouring agent capable of being added to a food product.

4. Flavouring composition comprising (ethoxy-1-ethenyl)-2-pyrroline-1.

5. Acetyl-2-pyrroline-1 being in encapsulated form capable of being obtained by the process in which, at a temperature of -10°C to 25°C, (ethoxy-1-ethenyl)-2-pyrroline-1 is hydrolysed with an acid, neutralisation is carried out with an equimolecular quantity of base, a cyclodextrin solution is added so as to obtain a solution containing up to 20 % by weight of acetyl-2-pyrroline-1 based on the cyclodextrin, and the said solution is then freeze-dried so as to obtain a powder which can contain up to 20 % by weight of acetyl-2-pyrroline-1.

6. Acetyl-2-pyrroline-1 being in encapsulated form capable of being obtained by the process in which, at a temperature of -10°C to 25°C, (ethoxy-1-ethenyl)-2-pyrroline-1 is hydrolysed with an acid, neutralisation is carried out with an equimolecular quantity of base, a maltodextrin solution is added so as to obtain a solution containing up to 10 % by weight of acetyl-2-pyrroline-1 based on the maltodextrin, and the said solution is freeze-dried so as to obtain a powder which can contain up to 10 % by weight of acetyl-2-pyrroline-1.

7. Use of the product according to claims 5 and 6, alone or in combination, as a flavour potentiator, capable of being added to a food product.

## Patentansprüche

1. (Ethoxy-1-ethenyl)-2-pyrrolin-1 mit der Formel:

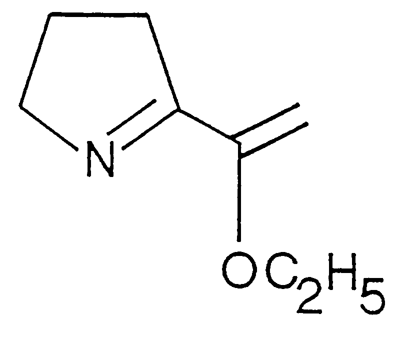

2. Verfahren zur Herstellung von (Ethoxy-1-ethenyl)-2-pyrrolin-1 nach Anspruch 1, wobei man bei einer Temperatur unter 0°C Ethylvinylether mit tert-Butyllithium in organischer Lösung reagieren läßt, N-Trimethylsilyl-pyrrolidinon-2 hinzufügt, es reagieren läßt, dann die erhaltene Lösung hydrolysiert, wobei man die organische Phase zurückgewinnt, die man trocknet und reinigt, um das gewünschte (Ethoxy-1-ethenyl)-2-pyrrolin-1 zu erhalten.

3. Verwendung von (Ethoxy-1-ethenyl)-2-pyrrolin-1 nach Anspruch 1 als Aromastoff, der dazu geeignet ist, einem Nahrungsmittel beigesetzt zu werden.

4. Aromazusammensetzung, die (Ethoxy-1-ethenyl)-2-pyrrolin-1 umfaßt.

5. Acetyl-2-pyrrolin-1 in verkapselter Form, erhältlich gemäß einem Verfahren, bei dem man bei einer Temperatur von -10°C bis 25°C (Ethoxy-1-ethenyl)-2-pyrrolin-1 mit einer Säure hydrolysiert, mit einer äquimolaren Menge Base neutralisiert, eine Cyclodextrin-Lösung hinzufügt, um eine Lösung zu erhalten, die bis zu 20 Gew.-% Acetyl-2-pyrrolin-1 im Verhältnis zu Cyclodextrin enthält und dann die Lösung lyophilisiert, um ein Pulver zu erhalten, das bis zu 20 Gew.-% Acetyl-2-pyrrolin-1 enthalten kann.

6. Acetyl-2-pyrrolin-1 in verkapselter Form, erhältlich gemäß einem Verfahren, bei dem man bei einer Temperatur von -10°C bis 25°C (Ethoxy-1-ethenyl)-2-pyrrolin-1 mit einer Säure hydrolysiert, mit einer äquimolaren Menge Base neutralisiert, eine Maltodextrinlösung hinzufügt, um eine Lösung zu erhalten, die bis zu 10 Gew.-% Acetyl-2-pyrrolin-1 im Verhältnis zu Maltodextrin enthält und dann die Lösung lyophilisiert, um ein Pulver zu erhalten, das bis zu 10 Gew.-% Acetyl-2-pyrrolin-1 enthalten kann.

7. Verwendung des Produkts nach den Ansprüchen 5 und 6, allein oder in Kombination, als Geschmacksverstärker, das man einem Nahrungsmittel zusetzen kann.
